# EUROPÄISCHE PATENTSCHRIFT

(11) **EP 0 490 159 B1**
(45) Veröffentlichungstag und Bekanntmachung des Hinweises auf die Patenterteilung: **11.10.1995**
(21) Anmeldenummer: 91120117.6
(22) Anmeldetag: 26.11.1991
(51) Int. Cl.: A61F 2/30

(54) **Vorrichtung zum Adaptieren der Länge einer Endoprothese für Röhrenknochen**
Length adaptor for long-bones endoprosthesis
Adaptateur d'allongement pour l'endoprothèse de l'os long

(30) Priorität: 07.12.1990 DE 4039064
(43) Veröffentlichungstag der Anmeldung: 17.06.1992
(73) Patentinhaber: ESKA Implants GmbH & Co., 23556 Lübeck (DE)
(72) Erfinder: Moser, Hans, W-6227 Oestrich-Winkel (DE)
(74) Vertreter: Fuchs Mehler Weiss

(56) Entgegenhaltungen:
- EP-A- 0 290 767
- FR-A- 2 267 080
- GB-A- 2 039 220
- GB-A- 2 137 884

## Beschreibung

Die Erfindung betrifft eine Vorrichtung zum Adaptieren der Länge einer Endoprothese für Röhrenknochen gemäß dem Oberbegriff des Anspruchs 1.

Bei bestimmten Indikationen, insbesondere Knochentumor, ist es oftmals notwendig, Röhrenknochen, wie beispielsweise den Femurknochen ganz oder teilweise zu ersetzen. Im Falle eines Totalersatzes wird der erkrankte Knochen entfernt und durch eine Totalendoprothese ersetzt, die im Falle des Femurknochens zwei Endoprothesegelenkteile, nämlich ein Hüftgelenksteil und ein Kniegelenksteil, aufweisen muß.

Eine solche Totalendoprothese ist beispielsweise bekannt geworden aus der DE 31 38 848 A1. Eine darin beschriebene Endoprothese ist zusammengefügt aus zwei Gelenkteilen und Zwischenstücken, wobei die Länge der Zwischenstücke je nach individuellen Patientenerfordernissen im Operationssaal ausgewählt werden kann, so daß die Länge der Endoprothese der Länge des zu entfernenden Knochens angepaßt werden kann.

Mit dieser Endoprothese sind postoperative Längenveränderungen, insbesondere bei noch im Wachstum befindlichen Patienten, nicht möglich. Lediglich ein erneuter schwerer Eingriff würde es gestatten, die einmal implantierte Endoprothese auszutauschen durch eine längere. Dies ist aus medizinischer Sicht nicht vertretbar.

Eine sogenannte Einrichtung zur Wiederherstellung der Extremitätenfunktion ist aus der G 81 08 959.7 U1 bekannt geworden. Diese Vorrichtung weist ein künstliches Kniegelenk sowie ein künstliches Hüftgelenk auf, von denen jedes mit jeweils einem von zwei teleskopisch miteinander verbundenen hülsenförmigen Teilen verbunden ist. Die hülsenförmigen Teile sind mittels einer Gewindespindel und einer an einem hülsenförmigen Teil fest angebrachten Gewindehülse durch Drehen der Gewindespindel gegeneinander längs verschieblich, die Vorrichtung also in ihrer Länge einstellbar. Die Vorrichtung kann während der Operation einmal in ihrer Länge eingestellt werden. Ein postoperatives Adaptieren der Länge der Vorrichtung ist nicht vorgesehen und nicht möglich, da keinerlei Einrichtung vorhanden ist, um nach der Implantation die beiden hülsenförmigen Teile zu verschieben.

Aus der DE 33 36 004 C1 ist ein Adapter für eine Knochen-Endoprothese bekannt geworden, der zwischen einem Gelenkteil und einem Prothesenschaftteil angeordnetist. Dieser Adapter ist getriebeartig aufgebaut derart, daß auf Betätigung eines Schraubenkopfes die hülsenartigen Adapterteile auseinander oder zueinander bewegt werden können. Hierbei ist bei einer Längenanpassung beispielsweise in der Wachstumsphase des Patienten kein Totalersatz der kürzeren Endoprothese nötig, sondern lediglich ein Schnitt vom Operateur auszuführen, um den besagten Schraubenkopf freizulegen und zu verdrehen, um den Adapter in seiner Länge den Gegebenheiten anzupassen.

Wenn diese Adapter auch schon Vorteile gegenüber dem modular aufgebauten Endoprothesen gemäß der weiter oben erwähnten Druckschriften aufweisen, so bleibt festzuhalten, daß zur Durchführung der Längenanpassung noch ein invasiver Eingriff vorgenommen werden muß, nämlich um das Getriebe des Adapters erreichen zu können. Dies wird als nachteilig empfunden, insbesondere im Hinblick auf eine mögliche Keimverschleppung, die im Falle des Ausbruchs einer Entzündung den Erfolg der Implantation der Endoprothese in Frage stellen kann.

Die aus der EP 0 290 767 A1 bekannt gewordene Endoprothese ist mit einem ganz ähnlichen Problem behaftet. Auch hier sind zwei teleskopisch ineinander führbare Hülsen vorgesehen, die mit weiteren Endoprothesenteilen wie Gelenkteil und Schaftteil verbindbar sind. Eine Hülse ist mit einem Außengewinde, die andere mit einem Innengewinde versehen. Die Längsverstellung der Endoprothese erfolgt über ein Verdrehen der Teile, wobei ein Kegelradgetriebe vorgesehen ist, dessen Antriebsspindel postoperativ von außen durch eine Stichinzision zugänglich gemacht werden kann.

Darüber hinaus sei auf die noch weiter fern liegende Vorrichtung gemäß der US-PS 4,863,473 hingewiesen, mit Hilfe derer das Skelett eines Verstorbenen nach Entnahme von Knochenteilen zu Transplantationszwecken optisch wieder hergerichtet werden kann.

Aus der gattungsbildenden GB-A-2137884 ist eine Wachstumsprothese bekannt, bei der zu ihrer Distraktion Kugeln in einen Kugelkanal eingebracht werden. Der Kugelkanal ist gebildet in zwei ineinander verschieblich gelagerten, kopfseitig geschlossen Hülsen. Nach Bedarf werden zusätzliche Kugeln in den Kugelkanal eingebracht, so daß der Adapter distrahiert wird.

Aus der FR-A-2 267 080 ist die Anwendung des hydraulischen Prinzips mit Kolben und Druckraum bei Adaptionsendoprothesen bekannt. Das Implantat ist vollständig implantierbar und weist eine Pumpe auf, mittels derer hydraulische Flüssigkeit in einen Kolbenraum geleitet wird, welche daraufhin den Kolben in Distraktionsrichtung drückt. Mit diesem Implantat ist die nichtinvasive Distraktion eines Gliedmaßes möglich.

Dieser Druckschrift ist allerdings keine Sicherheitsvorrichtung zu entnehmen für den Fall, daß die hydraulische Flüssigkeit aus dem System austritt und die Vorrichtung unter der Körperbelastung wieder in sich zusammengeschoben wird, was unter Umständen schlagartig geschehen kann, so daß der mit dem Implantat versorgte Patient einem Risiko der ruckartigen Kontraktion des Adapters ausgesetzt ist. Dies wird als nicht mehr hinnehmbar empfunden.

Aufgabe der vorliegenden Erfindung ist es daher, die gattungsgemäße Vorrichtung so weiterzubilden, daß sie eine später vorzunehmende längenanpassung ohne invasiven Eingriff möglich macht und daß die Vorrichtung gegenüber den Auswirkungen einer Leckage des Systems und damit gegenüber einer unkontrollierbaren Kontraktion gesichert ist.

Gelöst wird diese Aufgabe durch die kennzeichnenden Merkmale des Anspruchs 1. Die Außenwandung der inneren Hülse des Adapters ist mit mindestens einer Zahnleiste versehen, welche Zähne aufweist, deren ansteigende Zahnflanken in Richtung auf die zulässige Expansionsrichtung des Adapters geneigt sind.

Vorteilhaft ist hier ein Winkel zwischen diesen Zahnflanken und der Expansionsrichtung von ca. 30°.Die abfallenden Zahnflanken hingegen schließen mit der Expansionsrichtung einen Winkel von <90° ein.

Das Gegenstück zu einer solchen Zahnleiste befindet sich innerhalb mindestens einer Ausnehmung im Inneren der äußeren Hülse in Form einer Arretierungsbacke. Diese ist in der Ausnehmung unter Vorspannung in Richtung auf die innere Hülse des Adapters beweglich gelagert. Sie ist an ihre der inneren Hülse zugewandten Seite mit einer Zahnleiste versehen, die entsprechend der Zahnleiste an der Außenwandung der inneren Hülse ausgebildet, zu dieser allerdings umgekehrt angeordnet ist. Die Zahnleisten auf der inneren Hülse des Adapters und jene auf der Arretierungsbacke sind also miteinander verrastbar. Sie sind miteinander verrastet, wenn der Druckraum im Adapter drucklos ist. Dies ist dann der Fall, wenn auf die Betätigung der Pumpe hin eine Ausgleichsbewegung im Sinne einer Expansionsbewegung des Adapters beendet ist. Dies ist aber auch dann der Fall, wenn das System leckt. Der Adapter behält in diesem Fall also die zuletzt eingestellte Lage der Endoprothese bei, dadurch, daß die erwähnten Zahnleisten in fester Verrastung verharren.

Gleichwohl sind bei intaktem System aufgrund der Formgebung der Zahnleisten und unter Aufhebung der Vorspannung, mit der die Arretierungsbacke gegen die innere Hülse gedrückt wird Längeneinstellungen möglich.

Wird nämlich die Pumpe des Systems betätigt, also der Druckraum druckbeaufschlagt, bewirkt die Form der Zähne der Zahnleisten ein leichtes Abheben voneinander entgegen der besagten Vorspannung, so daß die Arretierungsbacke etwas in Richtung auf das Innere der sie lagernden Ausnehmung gedrückt wird. Ist der Druck im Druckraum groß genug, führt die äußere Hülse eine Ausgleichsbewegung in Expansionsrichtung aus, solange bis der Druck abgebaut ist, worauf die Zahnleisten wieder sofort in die sichere Verrastung übergehen.

Der Adapter weist in besonders bevorzugter Ausbildung an seiner inneren Hülse zwei gegenüberliegende Zahnleisten auf, die mit den Zahnleisten zweier Arretierungsbacken, welche in entsprechend zwei gegenüber liegenden Ausnehmungen in der äußeren Hülse gelagert sind, im beschriebenen Sinne zusammenwirken. Dieser Adapter ist von relativ einfachem mechanischen Aufbau, weist aber sämtliche beschriebenen Vorteile auf.

Die erwähnte Vorspannung der Arretierungsbacken in Richtung auf die innere Hülse des Adapters wird vorzugsweise von Puffern erzeugt, die aus körperverträglichem Silikon bestehen und zwischen den Arretierungsbacken und dem Boden der sie jeweils lagernden Ausnehmungen eingelassen sind.

Für die Voreinstellung der Länge des Adapters bzw. der ganzen Endoprothese vor der Implantation ist es zweckmäßig, die Arretierungsbacken mit einer Vorrichtung zu versehen, die ein zwangsweises Trennen der Zahnleisten voneinander ermöglicht. Hierbei sind die Arretierungsbacken vorzugsweise jeweils mit einer von außen zugänglichen Gewindebohrung versehen, in die eine Gewindeschraube geschraubt werden kann, um die Arretierungsbacken gegen den Boden der sie lagernden Ausnehmungen zu ziehen, wobei die Zahnleisten außer Eingriff kommen. Nach erfolgter Voreinstellung der Länge werden die Gewindeschrauben entfernt, so daß die erwähnte Verrastung der Zahnleisten eintritt.

Das generelle Prinzip der Verrastung gegeneinander beweglicher Teile eines Implantates ist im übrigenbeschrieben in der nachveröffentlichten DE 4012622 C1, die allerdings ein rein mechanisches Wirbelkörperimplantat beschreibt.

Als Druckflüssigkeit kommt vorzugsweise eine sterile Kochsalzlösung zur Anwendung. Selbst im Falle einer Leckage des Systems kommt es dann nicht zu Bioumverträglichkeiten.

Die erfindungsgemäße Vorrichtung gestattet - wie eingangs erwähnt - eine postoperative Längenverstellung im Sinne einer Expansion der mit ihrem Adapter verbundenen Endoprothese ohne invasiven Eingriff. Sie wird anhand eines Ausführungsbeispiels gemäß den Zeichnungen näher erläutert. Es zeigt:
- Fig. 1: eine Schnittansicht des prinzipiellen Aufbaus der erfindungsgemäßen Vorrichtung,
- Fig. 2: eine geteilte Schnittansicht durch den Adapter auf der Höhe A bzw. B gemäß Fig. 1, und
- Fig. 3: eine vergrößerte Ansicht der Einzelheit Z aus Fig. 1.

In den Figuren sind gleiche Teile mit denselben Bezugszeichen versehen.

Die Vorrichtung besteht aus dem Adapter 1 und der mit ihm über eine Druckleitung 10 in Verbindung stehenden manuell betätigbaren Pumpe 9, die aus einem Flüssigkeitsreservoir 8 Flüssigkeit in den Adapter 1 pumpen kann. Die Pumpe 9 besteht ebenso wie das Flüssigkeitsreservoir 8 aus körperverträglichem Silikon. Auch die Druckleitung 10 besteht - wie im übrigen auch die Versorgungsleitung zwischen dem Flüssigkeitsreservoir 8 und der Pumpe 9 - aus bioverträglichem Silikon.

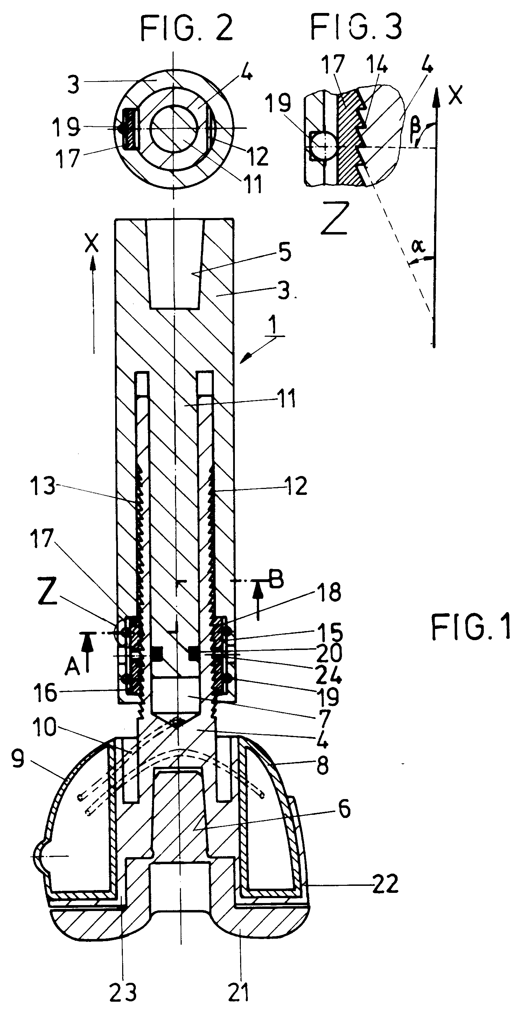

Der Adapter 1 ist zwischen zwei Endoprothesenteilen anzuordnen, von denen in Fig. 1 zur Veranschaulichung ein Gelenkteil 21 für ein Kniegelenk dargestellt ist, das die natürlich Kondylen des Femurteils des Gelenks durch Gleitkufen nachbildet.

Der Adapter 1 besteht aus zwei ineinander verschieblich gelagerten, kopfseitig geschlossenen Hülsen 3 und 4. Diese weisen in ihrem äußeren Kopfende jeweils eine konisch sich verjüngende Bohrung 5 bzw. 6 auf, in der die Endoprothesenteile mit entsprechend ausgebildeten Zapfen in einen Klemmsitz gebracht werden können. Dies ist für das Gelenkteil 21 in Fig. 1 angedeutet.

Die äußere Hülse 3 ist mit einem Kolben 11 versehen, der paßgenau in das Innere der inneren Hülse 4 ragt. Der Kolben 11 ist mit der Hülse 3 längsverschieblich in der Hülse 4 bewegbar.

Zwischen dem Kopf des Kolbens 11 und dem inneren Kopfende der Hülse 4 ist ein Druckraum 7 begrenzt, der über einen nicht dargestellten Anschlußstutzen mit der Druckleitung 10 verbunden ist. Der Druckraum 7 ist durch den Kolbenring 20, der im Kopfbereich des Kolbens 11 in diesen eingelassen ist, abgedichtet.

Das Flüssigkeitsreservoir 8, die Anschlußleitung zur Pumpe 9, die Pumpe 9 selbst, die Druckleitung 10 und schließlich der Druckraum 7 sind mit Flüssigkeit gefüllt und bilden ein entlüftetes, geschlossenes hydraulisches System. Wird der Druckraum 7 durch Betätigung der Pumpe 9 druckbeaufschlagt, so führt die Hülse 3 eine Ausgleichsbewegung in die Expansionsrichtung X aus.

Der Kopfteil der Hülse 4 ist vorliegend im übrigen derart an das spezielle Gelenkteil 21 angepaßt, daß es je eine Halterung 22 bzw. 23 für das Flüssigkeitsreservoir 8 bzw. für die Pumpe 9 aufweist. So ergibt sich eine kompakte implantierbare Einheit.

Wie aus den Fig. 1 und 2 deutlich zu erkennen ist, sind an der Außenwandung der inneren Hülse 4 zwei Zahnleisten 12 und 13 vorgesehen. Deren Zähne 14 sind, wie anhand Fig. 3 deutlich wird, so ausgebildet, daß die ansteigenden Zahnflanken in Richtung X der Expansionsbewegung geneigt sind, und zwar in einem Winkel α von ca. 30°. Die abfallenden Zahnflanken schließen einen Winkel β zur Richtung X ein, der < 90° sein muß.

Im Inneren der äußeren Hülse 3 sind zwei Ausnehmungen 15, 16 vorgesehen. In diesen ist jeweils eine Arretierungsbacke 18 bzw. 17 gelagert. Deren zur inneren Hülse 4 gewandte Stirnflächen weisen jeweils eine Zahnleiste auf, die entsprechend den Zahnleisten 13 und 14 an der inneren Hülse 3 ausgebildet, aber umgekehrt zu diesen angeordnet sind.

Im Boden jeder Ausnehmung 15 und 16 sind jeweils Puffer 19 aus Silikon eingelassen, welche die Arretierungsbacken 17 und 18 in Richtung auf die innere Hülse 3 drücken. Die Anordnung ist derart, daß die Ausnehmungen 15 und 16 mit den darin gelagerten Arretierungsbacken 18 bzw. 17 jeweils gegenüber einer Zahnleiste 12 bzw. 13 an der Außenwandung der inneren Hülse 3 liegen.

Wenn der Druckraum 7 drucklos ist, also auch keine der durch die Puffer 19 erzeugte Vorspannung der Arretierungsbacken 17 und 18 entgegengesetzte Kraft wirkt, sind die Zahnleisten der inneren Hülse 3 und jene der Arretierungsbacken in sicherer, aber lösbarer Verrastung. Diese bietet u.a. Sicherheit gegen eine unkontrollierte Kontraktion des Adapters 1 im Falle einer Leckage des hydraulischen Systems. Die Sicherheit bestimmt im übrigen entscheidend der bereits erwähnte Winkel β der abfallenden Zahnflanken mit. Wären auch Werte für β von > 90° zugelassen, könnte es zu einem Abrutschen der äußeren Hülse 3 kommen, wenn Belastungskräfte entgegen der Richtung X wirken. Aufgrund der Vorgabe aber, daß β < 90° sein muß, ist dies nicht möglich.

Dennoch ist trotz des innigen Eingriffs der Zahnleisten eine Expansion des Adapters möglich. Wird nämlich der Druck im Druckraum 7 erhöht durch Betätigen der Pumpe 9, wird eine die Vorspannung der Arretierungsbacken 17 und 18 teilweise aufhebende Kraft erzeugt, so daß die Verrastung der Zahnleisten teilweise aufgehoben wird. Die Ausgleichsbewegung der Hülse 3 in die Richtung X bewirkt eine Bewegung der Zahnleisten gegeneinander gewissermaßen Zahn um Zahn. Nach dem Druckabbau im Druckraum 7 kehrt das System automatisch in die sichere Verrastungsstellung zurück. Entscheidend für die Möglichkeit der Ausführung einer Bewegung in Richtung X der Expansion trotz inniger Verrastung der Zahnleisten im Ruhezustand des Adapters 1 ist die Wahl des Winkels α der ansteigenden Zahnflanken. Als vorteilhaft hat sich ein Wert für α von ca. 30° ergeben.

Jede der Arretierungsbacken 17 und 18 weist im übrigen eine Gewindebohrung 24 auf, in die durch eine Bohrung in der äußeren Hülse 3 eine Gewindeschraube (nicht dargestellt) geschraubt werden kann. Die Schraube soll so ausgebildet sein, daß sie sich mit ihrem Kopf an der Hülse 3 anlegt, wenn die Arretierungsbacken 17 und 18 unter Aufheben der Vorspannkraft von den Puffern 19 zwangsweise zum Boden der Ausnehmungen 16 bzw. 15 gezogen werden. Die Ausnehmungen 15 und 16 haben eine solche Tiefe, daß die Zahnleisten 12 und 13 und jene der Arretierungsbacken dann völlig außer Eingriff kommen. In dieser Lage kann der Adapter 1 vor der Implantation in seiner Länge voreingestellt werden.

## Patentansprüche

1. Vorrichtung zum Adaptieren der Länge einer Endoprothese für Röhrenknochen, mit einem Adapter (1), der zwischen einem mit ihm verbundenen Endoprothesengelenkteil und einem Endoprothesenschaftteil oder zwischen zwei Endoprothesengelenkteilen oder zwischen zwei Endoprothesenschaftteilen anzuordnen ist und aus zwei ineinander verschieblich gelagerten, kopfseitig geschlossen Hülsen (3, 4) aufgebaut ist, die an ihrem jeweiligen äußeren Kopfende eine Aufnahmevorrichtung (5, 6) für die mit dem Adapter (1) verbindbaren Endoprothesenteile aufweisen und von denen eine in ihrem Inneren einen Kolben (11) aufweist, der in dem Inneren der anderen Hülse (4) längsverschieblich bewegbar ist und zusammen mit dem inneren Kopfende der anderen Hülse (4) einen Raum einschließt, dadurch gekennzeichnet,
daß der Raum ein flüssigkeitsdichter und flüssigkeitsbefüllter Druckraum (7) ist, daß die Vorrichtung eine ein Flüssigkeitsreservoir (8) aufweisende, aus körperverträglichem Material bestehende Pumpe (9) aufweist, die mit dem Druckraum (7) über eine Druckleitung (10) in Verbindung steht, durch welche unter Pumpwirkung Flüssigkeit in den Druckraum (7) einleitbar ist, und
daß die Außenwandung der inneren Hülse (4) des Adapters (1) mit mindestens einer Zahnleiste (12, 13) mit Zähnen (14) versehen ist, deren ansteigende Zahnflanken in Richtung X der zulässigen Expansionsbewegung des Adapters geneigt sind und deren abfallende Zahnflanken einen Winkel von kleiner 90° zur Richtung X der Expansionsbewegung einschließen, und bei der Im Inneren der äußeren Hülse (3) mindestens eine Ausnehmung (15, 16) vorgesehen ist, innerhalb der eine Arretierungsbacke (17, 18) unter Vorspannung in Richtung auf die innere Hülse (4) beweglich gelagert ist, die eine entsprechend der Zahnleiste (13, 14) der inneren Hülse (4) ausgebildete und komplementär zu dieser angeordnete Zahnleiste aufweist, welche in drucklosem Zustand des Druckraums (4) mit jener verrastet ist, derart, daß eine Bewegung der äußeren Hülse (3) entgegen der Expansionsbewegung unmöglich ist, wobei der Eingriff der Zahnleisten bei Druckbeaufschlagung des Druckraumes (7) soweit entgegen der erwähnten Vorspannung aufgehoben wird, daß eine Bewegung der äußeren Hülse (3) in Expansionsrichtung X ermöglicht wird.

2. Vorrichtung nach Anspruch 1, bei der die Vorspannung der Arretierungsbacke (17, 18) von mindestens einem zwischen ihr und der Innenwandung der Ausnehmung (15, 16) angeordneten flexiblen Silikonpuffer (19) erzeugt wird.

3. Vorrichtung nach Anspruch 1 oder 2, bei der die innere Hülse (4) zwei gegenüberliegende Zahnleisten (12, 13) aufweist, die in drucklosem Zustand des Druckraumes (7) mit den Zahnleisten zweier Arretierungsbacken (17, 18), die in entsprechend zwei gegenüberliegenden Ausnehmungen (15, 16) in der äußeren Hülse (3) gelagert sind, verrastet sind.

4. Vorrichtung nach einem der Ansprüche 1 bis 3, bei der die Arretierungsbacke (17, 18) mit einer von außen zugänglichen Gewindebohrung (21) versehen ist, in die eine Gewindeschraube schraubbar ist, um die Zahnleiste der Arretierungsbacke (17, 18) zwangsweise entgegen der Richtung der Vorspannung außer Eingriff aus der Zahnleiste (12, 13) an der inneren Hülse (4) zu bringen.

5. Vorrichtung nach einem der Ansprüche 1 bis 4, bei der der Druckraum (7) gegenüber der Umgebung durch einen Kolbenring (20), der im Kopfbereich des Kolbens (11) angebracht ist, aus körperverträglichem Silikon abgedichtet ist.

6. Vorrichtung nach einem der vorhergehenden Ansprüche, bei der die Pumpenflüssigkeit eine isotonische Kochsalzlösung ist.

## Claims

1. Device for adapting the length of an endoprosthesis for long bones, having an adapter (1), which should be arranged between an endoprosthesis joint part connected to it and an endoprosthesis shaft part or between two endoprosthesis joint parts or between two endoprosthesis shaft parts, and is constructed from two sleeves (3, 4) closed on the head side and mounted displaceably in one another and which have a receiving device (5, 6) for the endoprosthesis parts which can be connected using adapter (1) on their particular outer head end, and of which one has a piston (11) in its interior, which can be moved longitudinally displaceably in the interior of the other sleeve (4), and encloses a chamber together with the inner head end of the other sleeve (4), characterised in that the chamber is a fluid-tight and fluid-filled pressure chamber (7), in that the device has a pump (9) consisting of body-compatible material and having a fluid reservoir (8), which pump (9) is connected to pressure chamber (7) via a pressure pipe (10) through which fluid can be introduced into pressure chamber (7) under pump action, and in that the outer wall of the inner sleeve (4) of adapter (1) is provided with at least one tooth rail (12, 13) having teeth (14), the ascending tooth flanks of which are inclined in direction X of the admissible expansion movement for the adapter, and the descending tooth flanks of which enclose an angle of less than 90° to the direction X of the expansion movement, and in which at least one recess (15, 16) is provided in the interior of the outer sleeve (3), within which recess (15, 16) an arresting jaw (17, 18) is movably mounted under pretension in the direction of inner sleeve (4) which has a tooth rail designed to correspond to the tooth rail (13, 14) of inner sleeve (4) and arranged to be complementary to the latter, and which in the pressure-free state of pressure chamber (4) is locked with it, such that a movement of the outer sleeve (3) counter to the expansion movement is impossible, wherein the engagement of the tooth rails is lifted counter to the said pretension when exposing pressure chamber (7) to pressure such that movement of the outer sleeve (3) is facilitated in expansion direction X.

2. Device according to claim 1, in which the pretension of arresting jaw (17, 18) is produced by at least one flexible silicone buffer (19) arranged between it and the inner wall of the recess (15, 16).

3. Device according to claim 1 or 2, in which the inner sleeve (4) has two opposing tooth rails (12, 13) which are locked in the pressure-free state of pressure chamber (7) with the tooth rails of two arresting jaws (17, 18), which are mounted correspondingly in two opposing recesses (15, 16) in the outer sleeve (3).

4. Device according to one of claims 1 to 3, in which the arresting jaw (17, 18) is provided with a threaded bore (21) accessible from the outside, into which a threaded screw can be screwed to forcibly disengage the tooth rail of arresting jaw (17, 18) from tooth rail (12, 13) on inner sleeve (4) counter to the direction of pretension.

5. Device according to one of claims 1 to 4, in which pressure chamber (7) is sealed from the surroundings by a piston ring (20) attached in the head region of piston (11) and made from body-compatible silicone.

6. Device according to one of the preceding claims, in which the pump fluid is an isotonic saline solution.

## Revendications

1. Dispositif pour adapter la longueur d'une endoprothèse pour des os longs, avec un adaptateur (1), qui doit être disposé entre une partie d'articulation d'endoprothèse qui lui est reliée et une partie de tige d'endoprothèse ou entre deux parties d'articulation d'endoprothèse ou entre deux parties de tige d'endoprothèse, et qui est constitué de deux douilles (3, 4) montées coulissantes l'une dans l'autre, fermées à leur extrémité de tête et présentant, à leur extrémité de tête extérieure respective, un dispositif récepteur (5, 6) pour les parties d'endoprothèse à relier à l'adaptateur (1), et dont l'une présente en son intérieur un piston (11) qui peut être déplacé en coulissement longitudinal à l'intérieur de l'autre douille (4) et qui délimite une chambre conjointement avec l'extrémité de tête intérieure de l'autre douille (4),
**caractérisé** en ce que la chambre est une chambre de pression (7) étanche aux liquides et remplie de liquide, en ce que le dispositif présente une pompe (9) présentant un réservoir de liquide (8), réalisée en matériau compatible avec le corps humain et reliée à la chambre de pression (7) par une conduite de pression (10) permettant d'introduire du liquide dans la chambre de pression (7) sous l'action de la pompe, en ce que la paroi extérieure de la douille intérieure (4) de l'adaptateur (1) est pourvue d'au moins une barrette dentée (12, 13) présentant des dents (14) dont les flancs de dents ascendants sont inclinés dans la direction X du mouvement admissible d'expansion de l'adaptateur et dont les flancs de dents descendants forment un angle inférieur à 90° avec la direction X du mouvement d'expansion, et en ce qu'au moins un évidement (15, 16) est prévu à l'intérieur de la douille extérieure (3), évidement à l'intérieur duquel un mars d'arrêt (17, 18) est monté mobile sous précontrainte en direction de la douille intérieure (4), mars qui présente une barrette dentée de configuration correspondante à la barrette dentée (13, 14) de la douille intérieure (4), disposée complémentairement à cette dernière et engagée par crantage avec elle à l'état exempt de pression de la chambre de pression (7), de telle sorte qu'un déplacement de la douille extérieure (3) à l'encontre du mouvement d'expansion est impossible, l'engrènement des barrettes dentées étant supprimé lorsque la chambre de pression (7) est alimentée en pression ainsi qu'à l'encontre de la précontrainte précitée, de telle sorte qu'un déplacement de la douille extérieure (3) dans la direction d'expansion X est rendu possible.

2. Dispositif selon la revendication 1, **caractérisé** en ce que la précontrainte du mors d'arrêt (17, 18) est produite par un tampon flexible en silicone (19), disposé entre le mors et la paroi intérieure de l'évidement (15, 16).

3. Dispositif selon la revendication 1 ou 2, **caractérisé** en ce que la douille intérieure (4) présente deux barrettes dentées opposées (12, 13) qui, à l'état exempt de pression de la chambre de pression (7), sont engagées par crantage avec les barrettes dentées de deux mors d'arrêt (17, 18), qui sont montés dans deux évidements opposés correspondants (15, 16) dans la douille extérieure (3).

4. Dispositif selon l'une des revendications 1 à 3, **caractérisé** en ce que le mors d'arrêt (17, 18) est pourvu d'un perçage fileté (21) accessible de l'extérieur, dans lequel peut être vissé un boulon fileté afin de forcer la barrette dentée du mors d'arrêt (17, 18), à l'encontre de la direction de précontrainte, à se désengager de la barrette dentée (12, 13) de la douille intérieure (4).

5. Dispositif selon l'une des revendications 1 à 4, **caractérisé** en ce que la chambre de pression (7) est rendue étanche vis-à-vis de l'environnement par une bague de piston (20) en silicone compatible avec le corps humain, qui est placée dans la région de tête du piston (11).

6. Dispositif selon l'une des revendications précédentes, **caractérisé** en ce que le liquide de pompage est de l'eau salée isotonique.
